# EUROPEAN PATENT APPLICATION

(11) **EP 4 080 214 A1**
(43) Date of publication of application: **26.10.2022**
(21) Application number: 21892243.3
(22) Date of filing: 04.11.2021
(51) Int. Cl.: G01N 33/558, G01N 33/58, G01N 33/68

(54) **RAPID DETECTION METHOD FOR ANTI-DRUG ANTIBODY**

(30) Priority: 16.11.2020 KR 20200152735
(71) Applicant: Boditech Med Inc., Chuncheon-si, Gangwon-do 24398 (KR)
(72) Inventor: CHOI, Eui Yul, Chuncheon-si Gangwon-do 24214 (KR); LEE, Yoon Suk, Chuncheon-si Gangwon-do 24414 (KR); LEE, Mi Sook, Chuncheon-si Gangwon-do 24239 (KR); CHON, Hyang Ah, Chuncheon-si Gangwon-do 24379 (KR); SAYYED , Danishmalik Rafiq, Chuncheon-si Gangwon-do 24311 (KR)
(74) Representative: De Vries & Metman
(86) International application number: PCT/KR2021/015934
(87) International publication number: WO 2022/103075

(57) **Abstract**

The present invention provides a detection method capable of accurately, simply, and rapidly quantifying an anti-drug antibody present in a sample. The detection method of the present invention comprises steps of (a) dissociating a complex of an anti-drug antibody and a drug by contacting a biological sample including the complex of the anti-drug antibody and the drug with acid; (b) forming labeled immune complex of drug-antibody-drug by contacting the sample in which the complex is dissociated with a composition in dry form including a drug bound with a signal generating means and a drug bound with a linker; and (c) detecting a signal generated from the labeled immune complex.

## Description

### [Technical Field]

The present invention relates to a rapid detection method of anti-drug antibodies for monitoring the anti-drug antibodies.

### [Background Art]

### 1. Need for Therapeutic Drug Monitoring (TDM)

Therapeutic drug monitoring (TDM) refers to a series of activities that provide feedback while continuously observing drug response for optimal drug treatment of individual patients. The TDM is mainly used to maximize the therapeutic effect of each patient by monitoring drug concentration trends to maintain effective concentrations in the blood while minimizing drug hypersensitivity reactions. Drugs that are the main targets of the TDM include some antibiotics, anti-epileptic drugs, and cardiotropic drugs with well-known therapeutic concentration ranges, and about 10,000 cases are performed annually. Therapeutic drug concentration monitoring for optimal drug therapy can bypass individual pharmacokinetic differences by adjusting the drug therapy based on the measured drug concentration data when administering the drug with a well-known therapeutic range of plasma concentration. Recently, with the development of analytical chemistry to measure drugs and metabolites thereof in body fluids and the introduction of clinical pharmacokinetics, therapeutic drug monitoring in individual patients has already become common clinically, and the need for research and clinical applications in new medical fields is gradually increasing.

The TDM is a useful service from an economic point of view, and it can be evaluated that there is sufficient pharmacological evidence as well. The large difference in the response effect between individuals even with the same dose is because both pharmacokinetic and pharmacodynamic variations are included. When adjusted to the same concentration, the pharmacokinetic variation is removed and only the pharmacodynamic variation is left, so that the effect can be predicted more accurately and precisely.

Although the TDM provides useful information on the appropriate dose and usage of a drug, it is necessary to apply the results of TDM when it is determined that the benefits exceed the risks by entirely considering the severity, the degree of toxicity expression, and the like of the patient's disease. Accordingly, it is advantageous to predict an individual's pharmacokinetic parameters using concentration and dosing information, and to determine appropriate dose and usage to achieve a target concentration.

Monitoring for anti-TNF drugs among biologics, where the need for TDM is emerging, is further described. Anti-TNF drug is an antibody against tumor necrosis factor α (TNF-α), which is a major inflammatory mediator that causes chronic inflammatory diseases such as rheumatoid arthritis and inflammatory bowel disease. Anti-TNF drug was first used in Crohn's disease and are now widely used even in ulcerative colitis. However, the anti-TNF drug has disadvantages that it is very expensive compared to other existing drugs and may cause opportunistic infections such as tuberculosis. In addition, it is known that the anti-TNF drugs has no therapeutic effect in about 1/3 of patients, and in the remaining patients, if the administration is continued, the effect decreases.

In addition, it is known that about 40% of patients with Crohn's disease who initially responded to anti-TNF treatment show a loss of response. As the cause of the decrease in the therapeutic effect, a decrease in the concentration of the anti-TNF drug and the formation of antibodies against the anti-TNF drug in the blood are well known. In the West, where the anti-TNF drugs were first used, the concentration of anti-TNF drugs and the presence or absence of the formation of antibodies against the anti-TNF drugs in the blood were measured to determine whether the dose or usage should be changed, the drug is changed to another drug, or the current treatment continues when the effect is decreased during treatment.

A recently published meta-analysis showed a high tendency for the treatment response to disappear when an antibody to the anti-TNF drug was formed and when the concentration of the anti-TNF drug in the blood was low. The Trough Concentration Adapted Infliximab Treatment (TAXIT) study published in 2015 set a trough level target (3 to 7 µg/mL) for inflammatory bowel disease patients treated with infliximab maintenance therapy, to adjust the dosing schedule or drug dose. In the results of the TAXIT study, there was no difference in clinical remission rate between a clinically based dosing group and a concentration based dosing group in a maintenance phase, but in an optimization phase, the clinical remission rate of Crohn's disease patients increased from 65.1% to 88.4% (p = 0.02).

Another study also showed that when the trough level of infliximab was maintained at 6-10 µg/mL in Inflammatory Bowel Disease (IBD) patients, the mucosal healing rate evaluated using an endoscope was 80-90%. As such, when infliximab is administered to inflammatory bowel disease patients, overseas countries are already actively using TDM and reflecting the test results in treatment. Many domestic clinicians are also raising the need for TDM in infliximab treatment for inflammatory bowel disease patients. However, it is difficult to implement the TDM test during actual treatment due to the cost problem and the delay from the test to obtaining the infliximab trough level result.

### 2. Need for monitoring anti-drug antibodies (ADA)

In order to maintain a stable and sustained therapeutic effect of a biologic agent, quantitative monitoring of pharmacokinetics and stability evaluation for the biologic agent is required. However, ADA induced by the immune response of the biologic agent may neutralize the target binding, alter the clearance rate, interfere with PK analysis that measures drug concentration in the blood circulation, or significantly affect toxicokinetic (TK) analysis.

Safety concerns serve as a major factor in the need for immune response evaluation. In other words, the main key is to accurately identify a subject that causes an immune response by developing an assay capable of sufficiently detecting ADA even in the presence of a drug. For this reason, efforts to develop ADA assays showing increased drug tolerance are continuing, and interest in various approaches is increasing. Unlike the quantitative PK assay, since the ADA assay does not have an actual reference standard, it is difficult to quantify the immune response. Instead, the ADA assay defines a positive result by using samples obtained from subjects who have not undergone drug treatment as a statistical basis, and evaluates a positive result by measuring response specificity.

### 3. Problems of currently implemented test methods

### 3-1. Limitations of ELISA method

Currently, most TDM tests take blood from a patient and quantitatively measure the concentration using enzyme-linked immunosorbent assay (ELISA). The ELISA requires a complex process of reacting various reagents in precise volumes for a specific time in a 96-well plate. For reasons such as the use of expensive equipment in the central laboratory of a large hospital or the need for an examination by a specialist, the examination takes a long time and is expensive, and it takes more than three weeks for the results to be reflected in the actual treatment. In addition, there is a problem in that the subject has the inconvenience to visit the hospital additionally for TDM examination before administering the drug and the cost burden is increased.

### 3-2. Limitations of ADA test method

In order to accurately quantify anti-drug antibodies (ADA), a technique for measuring both a free form and a bound form with the drug is required. Many of commercial products measure only the free form, and some products measure total ADA and use the ELISA method. In the latter case, the pretreatment step of the sample is required through several steps and takes a considerable amount of time, so that there is a high possibility that numerical differences between testers may occur. In particular, the greater the number of samples, the higher the risk of cross-contamination and test errors.

A generally used ADA detection method is a multivalent ADA bridging method between a biotin-labeled drug and an enzyme-labeled drug. This method is susceptible to endogenous drug interference (false negative ADA) and/or drug target interference (false positive ADA). That is, depending on the dissociation reaction of the immune complex of the drug and ADA, ADA is captured by an assay reagent. However, the pretreatment steps used to isolate the immune complex are difficult to perform accurately and precisely because there is no reference standard that can clearly replace an ADA scenario in vivo. Moreover, in the process of separating the binding antibody from the drug and capturing ADA, direct competition of the drug due to overlapping of epitopes, steric hindrance due to very proximal epitopes, or inhibition of reaction due to the avidity or affinity of ADA may cause problems in the accuracy of ADA detection. For this reason, assays such as acid or base dissociation, competition inhibition of 3-party binding partners and/or elimination of interfering factors, solid phase extraction (SPE), and ACE have been developed or devised.

### 4. Need for developing POCT diagnostic method

In order to reduce not only a decrease in the therapeutic effect or side effects that may occur due to the different responses of each patent to the treatment, but also the economic burden on the patient, customized treatment with appropriate treatment at an appropriate time through TDM is required. In the TDM test, the prior enzyme-linked immunosorbent assay cannot be an appropriate method for customized treatment due to long test time, high cost, complexity, and the like. Accordingly, there is a need for a test method which is standardized and inexpensive and enables quick decision-making at the time of treatment by quickly confirming the results at the treatment site.

A point-of-care test (POCT) is a test that may be used for diagnosis and treatment by quickly and easily performing various tests without pre-treatment of the test sample without complex laboratory equipment near the subject (patient) to maximize the medical or economic effect. Therefore, the POCT can maximize the effectiveness of customized treatment by immediately monitoring the concentration of therapeutic drug required for customized treatment at the site.

Currently, only one product (Promonitor Quick anti-IFX) has been developed as an ADA detection POCT product using the point-of-care method. This product can be used on whole blood. However, since this product uses a qualitative analysis method and tests only free form antibodies, not drug-bound form antibodies, its clinical usefulness is extremely limited. That is, since a drug-bound form and a free form exist in blood, both the drug-bound antibody and the free antibody must be measured for accurate detection of ADA. In addition, there is no POCT product capable of detecting ADA from immune complexes in the presence of drugs and testing ADA with increased tolerance to drugs.

### [Technical Problem]

An object of the present invention is to provide a rapid detection kit capable of quantifying anti-drug antibodies present in a sample by an accurate, simple, and rapid method.

Another object of the present invention is to provide a rapid detection kit capable of accurately measuring a level of anti-drug antibody by a simple method without being affected by a drug present in an analyte or a patient sample.

### [Technical Solution]

An aspect of the present invention provides a rapid detection method of an anti-drug antibody comprising steps of (a) dissociating a complex of an anti-drug antibody and a drug by contacting a biological sample including the complex of the anti-drug antibody and the drug with acid; (b) forming a labeled drug-antibody-drug immune complex by contacting the sample in which the complex is dissociated with a composition in dry form including a drug to which a signal generating means is bound and a drug to which a linker is bound; and (c) detecting a signal generated from the labeled immune complex.

The composition in dry form may further include a stabilizer, a buffer, and a surfactant. The total volume of the sample in which the complex is dissociated and the composition in dry form may be 200 µl or less. The detecting of the signal generated from the labeled immune complex may include loading the labeled immune complex into a lateral flow cartridge including the test line immobilized with the linker.

### [Advantageous Effects]

According to the present invention, it is possible to quantify an anti-drug antibody present in a sample by an accurate, simple, and rapid method. In addition, it is possible to accurately measure the amount of anti-drug antibody by a simple method without being affected by a drug present in an analyte or a patient sample. In addition, the present invention enables more accurate treatment through accurate determination of an anti-drug antibody that may be related to effective treatment of a drug.

### [Description of Drawings]

FIG. 1 is a schematic diagram of an anti-drug antibody detection assay according to an embodiment of the present invention.
FIG. 2A illustrates the application of a sample to a lateral flow cartridge according to an embodiment of the present invention, and FIGS. 2B and 2C illustrate the intensities of detected fluorescence signals.
FIG. 3 is a diagram comparing the prior method and an embodiment of the present invention based on steps.
FIG. 4 is a diagram illustrating detailed time required in the prior method and an embodiment of the present invention.
FIG. 5 is a calibration curve showing the concentration of an anti-drug antibody (anti-infliximab) according to a signal in an embodiment of the present invention.
FIG. 6 illustrates the results of comparing the recovery (recovery rate) according to a free form or a bound form of the anti-drug antibody (anti-infliximab) according to an embodiment of the present invention.

### [Best Mode for the Invention]

The present invention provides a rapid detection method of an anti-drug antibody comprising steps of (a) dissociating a complex of an anti-drug antibody and a drug by contacting a biological sample including the complex of the anti-drug antibody and the drug with acid; (b) forming a labeled drug-antibody-drug immune complex by contacting the sample in which the complex is dissociated with a composition in dry form including a drug bound with a signal generating means and a drug bound with a linker; and (c) detecting a signal generated from the labeled immune complex.

In the present invention, the term "drug" refers to a drug manufactured from a raw material or material derived from a human or other organism, and includes all biological agents having effects such as prevention, treatment, improvement, delay, etc. for a specific disease. Examples of the drug in the present invention may include tumor necrosis factor α (TNF-α) inhibitors such as etanercept, infliximab, adalimumab, golimumab, and certolizumab pegol; B cell surface protein (CD20) binding monoclonal antibodies such as rituximab; vascular endothelial growth factor (VEGF) inhibitors such as bevacizumab; human epidermal growth factor receptor type 2 (HER2) inhibitors such as trastuzumab; integrin α4β7 inhibitors such as vedolizumab; interleukin 12 inhibitors such as ustekinumab; interleukin 17A inhibitors such as secukinumab; interleukin 6 inhibitors such as tocilizumab; human IgE inhibitors such as omalizumab, and the like.

In the present invention, the anti-drug antibody includes all human antibodies that specifically respond to the drugs listed above. Examples of the anti-drug antibody in the present invention may include all antibodies against the listed drugs, such as antietanercept antibody, anti-infliximab antibody, anti-adalimumab antibody, antigolimumab antibody, anti-rituximab antibody, anti-bevacizumab antibody, antivedolizumab antibody, anti-trastuzumab antibody, and the like.

The biological sample in the present invention is extracted from the human and may include liquids or liquid-like fluids such as blood, blood cells, lymph, saliva, urine, sweat, interstitial or intracellular body fluid, or materials extracted therefrom. The blood may include whole blood, plasma, serum, and the like, with or without a predetermined treatment (e.g., coagulation prevention), but is not limited thereto. The biological sample may be used in a manipulated or non-manipulated state.

In the present invention, the term "linker" refers to a compound capable of binding an antibody and a drug, and may be at least one selected from the group consisting of streptavidin, neutravidin, avidin, biotin, protein A/G, glutathione, a protein containing a histidine functional group, and an anti-histidine antibody.

The signal generating means in the present invention is a means for generating a signal by binding to antigens, antibodies, or proteins, and may include enzymes or enzyme proteins such as horseradish peroxidase (HRP), alkaline phosphatase (ALP), and glucose oxidase (GO); organic derivative fluorescent dyes such as xanthene derivatives, cyanine derivatives, squaraine derivatives, naphthalene derivatives, coumarin derivatives, oxidazole derivatives, anthracene derivatives, arylmethine derivatives, and dipyrromethene derivatives; inorganic derivative fluorescent dyes such as CdS/ZnS Quantum dot, CdSe Quantum dot, CdSe/ZnS Quantum dot, CdSSe/ZnS Quantum dot, CdTe/CdSe/ZnS Quantum dot, CdS Quantum dot, ZnCdSe/ZnS Quantum dot, CdTe Quantum dot, Eu, and Tb; fine particles such as latex and polystyrene colored with fluorescent dyes; and nanoparticles consisting of Au, Ag, Fe, Pt, Co or combinations thereof, but is not limited thereto.

In the present invention, the composition in dry form may include a drug bound with the signal generating means and a drug bound with the linker, and a stabilizer, a buffer and a surfactant as additives. The composition in dry form may be prepared by freeze drying or vacuum drying, and includes a powder or granular composition.

The stabilizer is a pharmaceutically acceptable excipient that protects the drug from chemical and/or physical degradation while preparing the composition in dry form. The stabilizer includes, for example, saccharides, amino acids (e.g., L-arginine, L-glutamic acid, and L-isoleucine), polyols (e.g., mannitol, sorbitol, xylitol, dextran, glycerol, arabitol, and propylene glycol), cyclodextrins (e.g., hydroxypropyl-β cyclodextrin, sulfobutylethyl-β cyclodextrin, and β cyclodextrin), polyethylene glycols (e.g., PEG 3000, PEG 3350, PEG 4000, PEG 6000, PEG 8000, and PEG 10,000), polyvinyl alcohol, polyvinylpyrrolidone, albumins (e.g., human serum albumin (HSA), bovine serum albumin (BSA), and ovalbumin) and salts (e.g., sodium chloride, magnesium chloride, and calcium chloride).

The saccharides include monosaccharides and oligosaccharides. The monosaccharides are monomeric carbohydrates that are not hydrolyzable by acids, such as monosaccharides and derivatives thereof, such as amino sugars. Examples of monosaccharides include glucose, lactose, fructose, galactose, mannose, sorbose, deoxyribose and neuraminic acid. The oligosaccharides are branched or linear carbohydrates made up of more than one monomeric saccharide units linked via glycosidic bonds. The monomeric saccharide units in the oligosaccharide may be the same as or different from each other. Depending on the number of monomeric saccharide units, the oligosaccharides may be di-, tri-, tetra-, penta-saccharides, and the like. Examples of the oligosaccharides include sucrose, trehalose, lactose, maltose and raffinose.

The buffer is a pharmaceutically acceptable salt that stabilizes the pH of a pharmaceutical agent. The buffer may be, for example, a citrate salt, an acetate salt, a histidine salt, a succinate salt, a maleate salt, a phosphate salt or a lactate salt. Specifically, the buffer includes Tris, HEPBS, HEPES, BIS-TRIS propane, phosphate, alkaline borate, Glycine, CAPSO, CHES, AMPSO, TABS, AMPD, TAPS, BICINE, Tricine, HEPPSO, POPSO, and Gly-Gly.

The surfactant is a pharmaceutically acceptable surfactant. The surfactant includes, for example, polyoxyethylene-sorbitan fatty acid ester (Tween), polyethylene alkyl ether (Brij), alkylphenylpolyoxyethylene ether (Triton X), polyoxyethylenepolyoxypropylene copolymer (poloxamer and pluronic), octylphenoxypolyethoxyethanol (IGEPAL CA-630) and CHAPS. Preferred polyoxyethylene-sorbitan fatty acid esters are polysorbate 20 (polyoxyethylene sorbitan monolaurate, Tween 20) and polysorbate 20 (polyoxyethylene sorbitan monooleate, Tween 80). Preferred polyethylene-polypropylene copolymers are those sold as Pluronic F68 or Poloxamer 188. Preferred polyoxyethylene alkyl ethers are those sold as Brij (trade name). Preferred alkylphenylpolyoxyethylene ether is commercially available as Triton X, preferably p-tert-octylphenoxy polyethoxyethanol (Triton X 100).

The present invention provides a rapid quantitative detection method of an anti-drug antibody present in patients administered with a therapeutic drug. The present invention is based on lateral flow assay that can easily provide a method for immunological analysis of immune complexes. The present invention provides a method for immunological determination of a complex in a sample using a sandwich type or double antigen bridging immunogenicity assay.

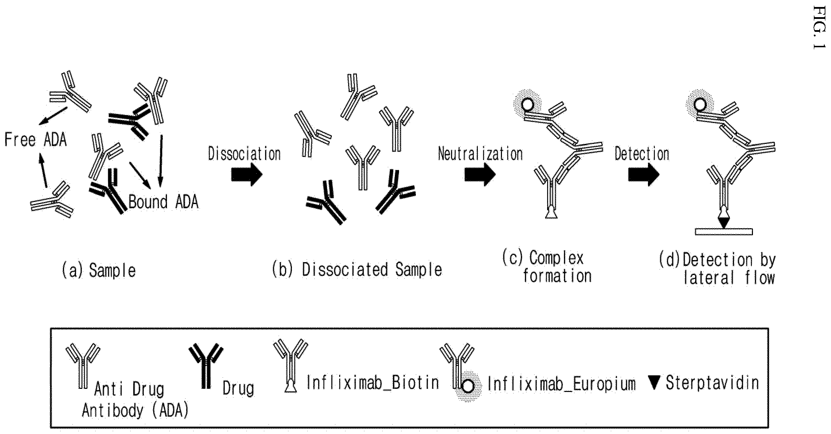

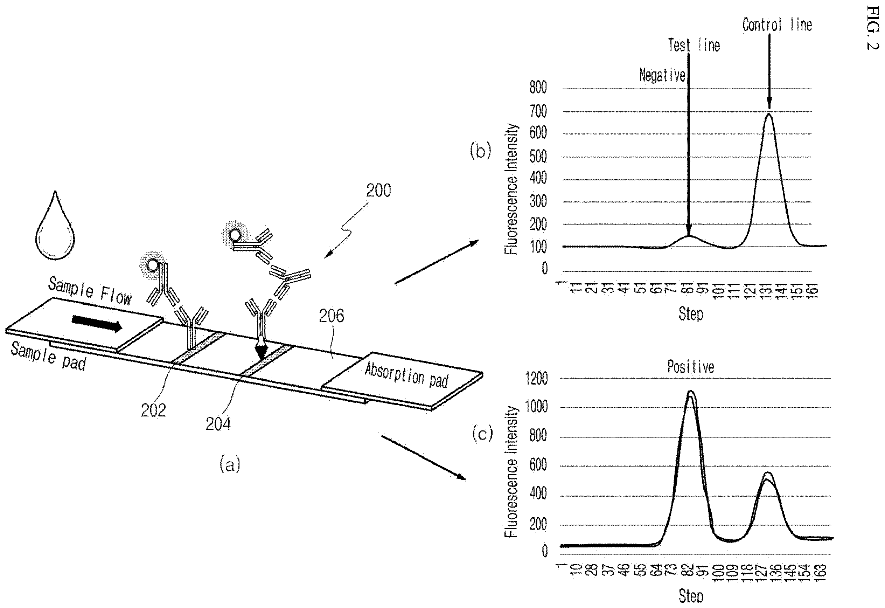

FIG. 1 is a schematic diagram of an anti-drug antibody detection assay according to an embodiment of the present invention. As illustrated in FIG. 1, a quantitative detection method according to the present embodiment comprises dissociation, neutralization, and detection steps. FIG. 2A illustrates the application of a sample to a lateral flow cartridge according to an embodiment of the present invention, and FIGS. 2B and 2C illustrate the intensities of detected fluorescence signals. A lateral flow cartridge 200 includes a sample pad 201, a control line 202, a test line 204, an absorption pad 205, and a porous membrane 206.

### Step 1: Dissociation step

From a drug-administered patient, a biological sample is extracted. As illustrated in FIG. 1A, an anti-drug antibody present in the extracted biological sample is present in a free form without binding to the drug or a bound form bound with the drug. In order to measure the exact level of the anti-drug antibody in the biological sample, as illustrated in FIG. 1B, the anti-drug antibody in the bound form needs to be dissociated into the anti-drug antibody in the free form. In the dissociation step, the biological sample is contacted with a dissociation buffer including an amount of acid sufficient to dissociate the anti-drug antibody in the bound form. Here, the acid includes an organic acid or an inorganic acid. The dissociation step requires 10 minutes or less, preferably 5 minutes or less, more preferably 2 minutes or less.

For prior ELISA methods, the preferred sample type is human plasma or human serum. Performing with human whole blood may give inconsistent results. However, in the present invention, consistent results can be obtained using not only human plasma and human serum, but also human whole blood.

### Step 2: Neutralization step

By contacting the sample in which the complex has been dissociated in Step 1 with a composition in dry form including the drug bound with the signal generating means and the drug bound with the linker, as illustrated in FIG. 1C, a labeled immune complex of drug-antibody-drug is formed. The composition in dry form may be prepared by mixing all of the buffer, the drug bound with the signal generating means and the drug bound with the linker and then drying them, or may be prepared by preparing each in dry form and then mixing. The buffer may further include additives that help to improve the solubility and the protein stability of the buffer. The neutralization step requires 20 minutes or less, preferably 10 minutes or less, more preferably 7 minutes or less.

In the prior general ELISA method, an additional operation of mixing the drug bound with the signal generating means, the drug bound with the linker, and the buffer and a container for storing each drug and buffer are additionally required. In contrast, in the present invention, since the composition in dry form is used, the drug bound with the signal generating means, the drug bound with the linker, and the buffer may be stored together in one storage container, so that there is an advantage that the operation steps are decreased to reduce the entire operation time and reduce the number of storage containers.

In addition, in the present invention, in order to reduce the time required in the neutralization step, nanoparticles are used as the signal generating means. The nanoparticles have a large surface area to bind to many drugs, thereby increasing immune reactivity and sensitivity. In addition, the nanoparticles bind to many drugs to increase the molecular size and increase their mobility, thereby increasing the reaction rate.

In the case of the prior ELISA method, a reaction or culture volume is 300 µl or more. On the other hand, in the case of the present invention, a culture volume is reduced to 200 µl or less and the concentration of the culture solution is increased to increase immune response kinetics, thereby performing the neutralization step within a short time so as to avoid the interference with endogenous drugs other than the target drug present in the sample. The culture volume may be 200 µl or less, preferably 150 µl or less, and more preferably 100 µl or less.

Unlike the prior method, the present invention reduces the reagent mixing step by drying the assay reagent in the neutralization and reaction step. In addition, the present invention avoids the interference effect of other endogenous drugs other than the target drug present in the sample which is the ultimate goal, by increasing the concentration of the assay reagent, reducing the volume of the reaction reagent, and using particles as a signal generating means to improve reaction sensitivity and reaction rate and minimize the time required in the neutralization step.

### Step 3: Detection step

The sample including the immune complex formed in Step 2 is put into the lateral flow cartridge as illustrated in FIG. 1D. Known lateral flow cartridges may be used. Based on the signal detected from the linker-fixed test line included in the lateral flow cartridge, a calibration curve is created using a pre-made formula to perform quantitative analysis. The detection step requires 20 minutes or less, preferably 15 minutes or less, more preferably 12 minutes or less.

In the series of steps, the user may directly transfer the sample formed in each step to the next step using a pipette or the like, or use a device or tool that automatically transfers the sample to the next step. Preferably, the series of steps may be performed automatically using the AFIAS series of Boditech Med Inc.

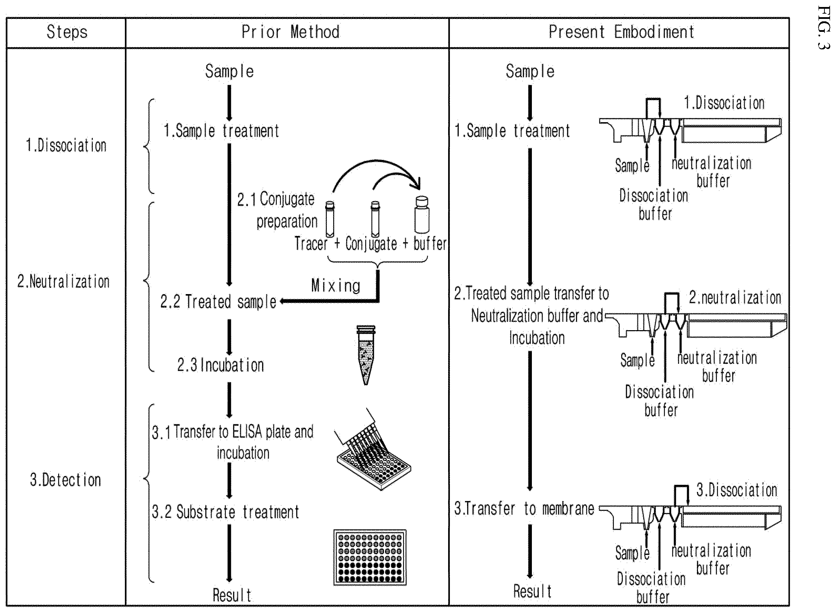

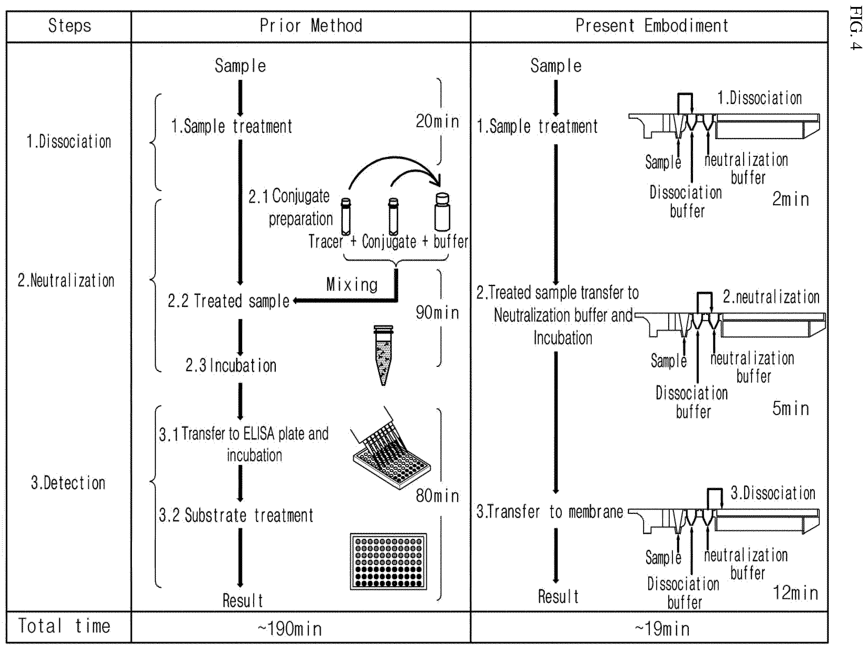

The present invention is very simple, and the number of steps is small compared to the prior ELISA method. Unlike the prior ELISA method, which includes at least 5 to 6 steps such as dissociation, conjugate preparation, neutralization, multiple incubation, and detection, the present invention may measure the amount of anti-drug antibody by using three steps of dissociation, neutralization, and detection (see FIG. 3). As described above, the present invention takes much less time than the prior ELISA method due to the minimization of operation steps. For operation completion and result generation, the prior ELISA method requires 3 to 12 hours, but the present invention requires less than 0.3 hour (see FIG. 4). Since the present invention is based on the lateral flow technique, there is no need for a washing operation generally involved in the ELISA method. For prior ELISA kits, the special care and a lot of time are required to produce reproducible results in the washing step. In addition, although the prior method requires special calculation for result analysis and quantification, the present method may automatically perform quantification using a pre-made formula.

Hereinafter, Examples of the present invention will be described in detail with reference to the accompanying drawings. However, the following Examples are just illustrative of the present invention, and the scope of the present invention is not limited to the following Examples.

In particular, the following Examples describe detailed procedures used to detect an anti-drug antibody and a drug immune complex using infliximab and anti-infliximab. The present invention may also detect other anti-drug antibodies in a similar manner. Accordingly, the present invention is never limited to the following Examples.

### Examples

### 1. Preparation of reagents

### 1-1. Dissociation buffer

A dissociation buffer was 0.1 M glycine of pH 1.92.

### 1-2. Composition in dry form used in neutralization step

1M Tris-HCl buffer containing 10% Tween-20, 5% BSA, and 1% poloxamer F68 was adjusted to pH 8.5. A neutralization buffer was prepared by adding 500 mM arginine and 150 mM MgCl₂ to the prepared buffer. The prepared neutralization buffer was dispensed in small amounts of 30 µl into each tube and freeze-dried for 4 days.

50 mg/mL NHS (n-hydroxysuccinimide) and 10 mg/mL EDC (1-(3-dimethylaminopropyl)-3-ethylcarbodiimide hydrochloride) were dissolved in DDW (double distilled water). Next, 230 µl of NHS solution and 239 µl of EDC solution were mixed with MES (2-(N-morpholino) ethane sulfonic acid) buffer (pH 6.1) containing 100 µl of europium nanoparticles to prepare 1 mL of a mixed solution. This mixed solution was mixed at 900 rpm at room temperature for 30 minutes, centrifuged at 14,000 rpm for 15 minutes, and the supernatant was removed. MES was then added, and the precipitate was dispersed by ultrasonication. 400 µl of 0.5 mg/mL infliximab antibody was mixed with the precipitate-dispersed solution and stirred at room temperature for 1 hour to bind the antibody to the nanoparticles. After repeating the centrifugation process three times as above, 50 mM Tris-HCl buffer containing 1% BSA was added to the recovered precipitate and dispersed to prepare a reagent containing a drug bound with the signal generating means.

After dissolving 0.1 M NaHCO₃ in the infliximab antibody, an appropriate amount of biotin-NHS (N-hydroxysuccinimide) ester was added to react. A reagent containing a drug bound with the linker was prepared by using size exclusion chromatography on the reacted solution.

Each of the reagent containing the drug bound with the prepared signal generating means and the reagent containing the drug bound with the linker was diluted with a Tris-HCl buffer containing 5% BSA, 3% sucrose, 1% PEG, and 0.1% PVA. Next, using a granule dispenser and a freeze dryer, dry granules were prepared.

The prepared dry granules and the composition obtained by drying the neutralization buffer were put in a tube and mixed to prepare a dry composition.

### 2. Preparation of lateral flow membrane

A lateral flow membrane 206 including a control line 202 containing ChIgY and a test line 204 containing streptavidin was prepared.

### 3. Preparation of samples

### i) Reference sample (free form)

An anti-infliximab antibody (Biorad) at a concentration of 1 mg/mL was serially diluted with human serum (from a healthy human) to prepare anti-infliximab antibody solutions at concentrations of 0 ng/mL, 10 ng/mL, 50 ng/mL and 250 ng/mL.

### ii) Reference sample (complex)

An anti-infliximab antibody (Biorad) at a concentration of 1 mg/mL was serially diluted with human serum (from a healthy human) to prepare anti-infliximab antibody solutions at twice concentrations of 0 ng/mL, 10 ng/mL, 50 ng/mL and 250 ng/mL. Next, the prepared anti-infliximab antibody solutions were mixed with 100 µg/ml of infliximab in a 1:1 ratio (v/v) to prepare anti-infliximab antibody solutions at concentrations of 0 ng/mL, 10 ng/mL, 50 ng/mL and 250 ng/mL including 50 µg/ml of infliximab.

### 4. Immunoassay

A cartridge was prepared in which the dissociation buffer prepared in the above step was added to the cartridge well, the tube containing the composition in dry form was inserted into the holder of the cartridge, and the tube inlet was sealed with aluminum. The immunoassay procedure was then performed automatically after adding the sample to the sample well and inserting the cartridge into the AFIAS (manufactured by Boditech Med Inc.) assay device.

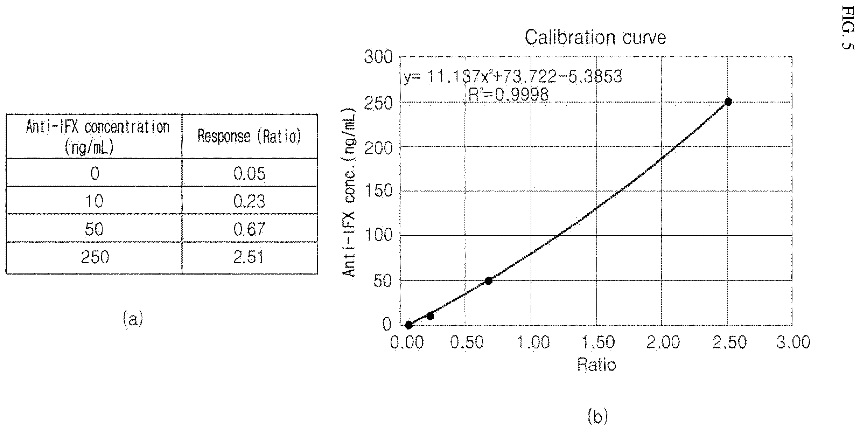

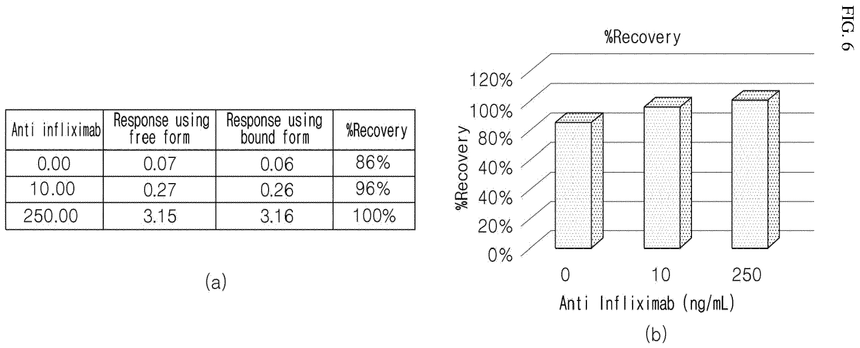

(Automatic performing process) 50 µl of a biological sample was added to a dissociation buffer well containing 300 µl of the dissociation buffer, mixed well and waited for 2 minutes. After the dissociation reaction was completed, 150 µl of the dissociated sample was transferred to the next well (well containing the composition in dry form), mixed well and waited for 5 minutes. After incubation, 75 µl of the mixture was transferred to the lateral flow membrane 206. After 12 minutes, the signal detected in the test line 204 was analyzed to obtain a result. The result was shown in FIGS. 5 and 6.

FIG. 5 illustrates signal ratio according to a concentration of anti-Infliximab (see FIG. 5A) and a calibration curve of the concentration of anti-Infliximab versus signal ratio (see FIG. 5B). FIG. 6 illustrates a result of an embodiment of the present invention, which illustrates values of comparing the recovery rate according to a free form or a bound form of the anti-drug antibody (anti-infliximab) (see FIG. 6A) and a graph (see FIG. 6B). The ratios shown in FIGS. 5 and 6 represent values obtained by dividing the intensity of the signal detected from the test line 204 by the intensity of the signal detected from the control line 202.

While the present invention has been particularly illustrated and described with reference to preferred embodiments thereof, it will be understood by those skilled in the art that various changes in forms and details may be made without departing from the scope of the present invention included in the appended claims. Also, it should be understood that the embodiments described herein are not mutually exclusive, and features of various embodiments may be combined in whole or in part according to the present invention.

## Claims

1. A rapid detection method of an anti-drug antibody comprising following steps of:
(a) dissociating a complex of an anti-drug antibody and a drug by contacting a biological sample including the complex of the anti-drug antibody and the drug with acid;
(b) forming labeled immune complex of drug-antibody-drug by contacting the sample in which the complex is dissociated with a composition in dry form including a drug bound with a signal generating means and a drug bound with a linker; and
(c) detecting a signal generated from the labeled immune complex.

2. The rapid detection method of the anti-drug antibody of claim 1, wherein the composition in dry form further includes a stabilizer, a buffer, and a surfactant.

3. The rapid detection method of the anti-drug antibody of claim 2, wherein the total volume of the sample in which the complex is dissociated and the composition in dry form is 200 µl or less.

4. The rapid detection method of the anti-drug antibody of any one of claims 1 to 3, wherein the detecting of the signal generated in the labeled immune complex includes loading the labeled immune complex into a lateral flow cartridge including the test line immobilized with the linker.
